# EUROPEAN PATENT APPLICATION

(11) **EP 2 613 291 A1**
(43) Date of publication of application: **10.07.2013**
(21) Application number: 12161822.7
(22) Date of filing: 28.03.2012
(51) Int. Cl.: G06T 3/40

(54) **Method and apparatus for displaying medical image**

(30) Priority: 04.01.2012 KR 20120001149
(71) Applicant: Samsung Medison Co., Ltd., Nam-myun Hongchun-gun Kangwon do 250-875 (KR)
(72) Inventor: Kim, Ho-young, 250-875 Gangwon-do (KR)
(74) Representative: Schmid, Wolfgang

(57) **Abstract**

A method of displaying a medical image, the method including: displaying a first image reduced from the medical image; determining a first point that is a location in the first image; determining a second point that is a location corresponding to the first point in a second image, wherein the second image is reduced from the medical image having a size greater than that of the first image; overlaying an image corresponding to a second region of a previously determined size including the second point over the first image; and receiving an input to move the second point and newly determining the second point based on the input.

## Description

### CROSS-REFERENCE TO RELATED PATENT APPLICATION

This application claims the benefit of Korean Patent Application No. 10-2012-0001149, filed on January 4, 2012, in the Korean Intellectual Properly Office, the disclosure of which is incorporated herein in its entirety by reference.

### BACKGROUND OF THE INVENTION

### 1. Field of the Intention

The present invention relates to a method and apparatus for displaying a medical image to select an accurate point in the medical image displayed on the apparatus.

### 2. Description of the Related Art

A medical diagnosis apparatus enables to acquire a medical image of a predetermined part of an internal of a subject and display the medical image in order for a user to diagnose the subject. The medical diagnosis apparatus is used for a medical purpose, such as detecting impurities from the internal of the subject, examine and observer injury thereof.

### SUMMARY OF THE INTENTION

The present invention provides a method and apparatus for displaying a medical image to acquire an accurate measurement value of the medical image displayed on the apparatus. The present invention also provides a computer readable recording medium having recorded thereon a program for executing the method. The technical objects of the present invention are not limited thereto and include other objects.

According to an aspect of the present invention, there is provided a method of displaying a medical image, the method including: displaying a first image reduced from the medical image; determining a first point that is a location in the first image; determining a second point that is a location corresponding to the first point in a second image, wherein the second image is reduced from the medical image having a size greater than that of the first image; overlaying an image corresponding to a second region of a previously determined size including the second point over the first image; and receiving an input to move the second point and newly determining the second point based on the input.

The overlaying may include overlaying the image corresponding to the second region over a first region including the first point of the first image.

The method may further include: newly determining the first point according to the newly determined second point; and overlaying an image corresponding to a new second region including the newly determined second point over a new first region including the newly determined first point.

The method may further include: marking the newly determined first point in the first image.

The method may further include: storing the newly determined second point.

The first image may include a plurality of display points present in a plurality of previously determined locations, wherein the overlaying includes: overlaying the image corresponding to the second region over a display region including one of the plurality of display points farthest away from the first point.

The input to move the second point may be received from a user using a mouse, a track ball, or a touch screen.

According to another aspect of the present invention, there is provided a method of displaying a medical image, the method including: displaying a reduction image reduced from the medical image; determining a first point that is a location in the reduction image; determining a second point that is a location corresponding to the first point in the medical image; overlaying an image corresponding to a second region of a previously determined size including the second point over the reduction image; and receiving an input to move the second point and newly determining the second point based on the input.

According to another aspect of the present invention, there is provided an apparatus for displaying a medical image, the apparatus including: a display unit for displaying a first image reduced from the medical image; a control unit for a determining a first point that is a location in the first image, and determining a second point that is a location corresponding to the first point in a second image, wherein the second image is reduced from the medical image having a size greater than that of the first image; and an input unit for receiving an input to move the second point, wherein the display unit overlays an image corresponding to a second region of a previously determined size including the second point over the first image, and wherein the control unit newly determines the second point based on the input.

According to another aspect of the present invention, there is provided an apparatus for displaying a medical image, the apparatus including: a display unit for displaying a reduction image reduced from the medical image; a control unit for determining a first point that is a location in the reduction image, and determining a second point that is a location corresponding to the first point in the medical image; and an input unit for receiving an input to move the second point, wherein the display unit overlays an image corresponding to a second region of a previously determined size including the second point over the reduction image, and wherein the control unit newly determines the second point based on the input.

According to another aspect of the present invention, there is provided a computer readable recording medium having recorded thereon a program for executing the method of displaying a medical image.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of an apparatus for displaying a medical image according to an embodiment of the present invention;
FIG. 2 is a flowchart of a method of displaying a medical image, according to an embodiment of the present invention;
FIG. 3 illustrates a medical image displayed on a display unit of an apparatus for displaying the medical image, according to an embodiment of the present invention;
FIG. 4 illustrates a medical image displayed on a display unit of an apparatus for displaying the medical image, according to another embodiment of the present invention;
FIG. 5 illustrates a medical image displayed on a display unit of an apparatus for displaying the medical image, according to another embodiment of the present invention; and
FIG. 6 illustrates a medical image displayed on a display unit of an apparatus for displaying the medical image, according to another embodiment of the present invention.

### DETAILED DESCRIPTION OF THE INVENTION

Hereinafter, preferred embodiments of the present invention will be described with reference to the attached drawings.

In the present specification, a "medical image" includes all images used for a medical purpose. For example, the "medical image" may include an ultrasound image, an X-ray image, a computed tomography (CT) image, or a magnetic resonance imaging (MRI) image. A "medical image apparatus" indicating all types of apparatuses for acquiring or displaying the above medical image may include an ultrasound diagnosis apparatus, an X-ray diagnosis apparatus, or special medical equipment such as a CT apparatus, an MRI apparatus, etc.

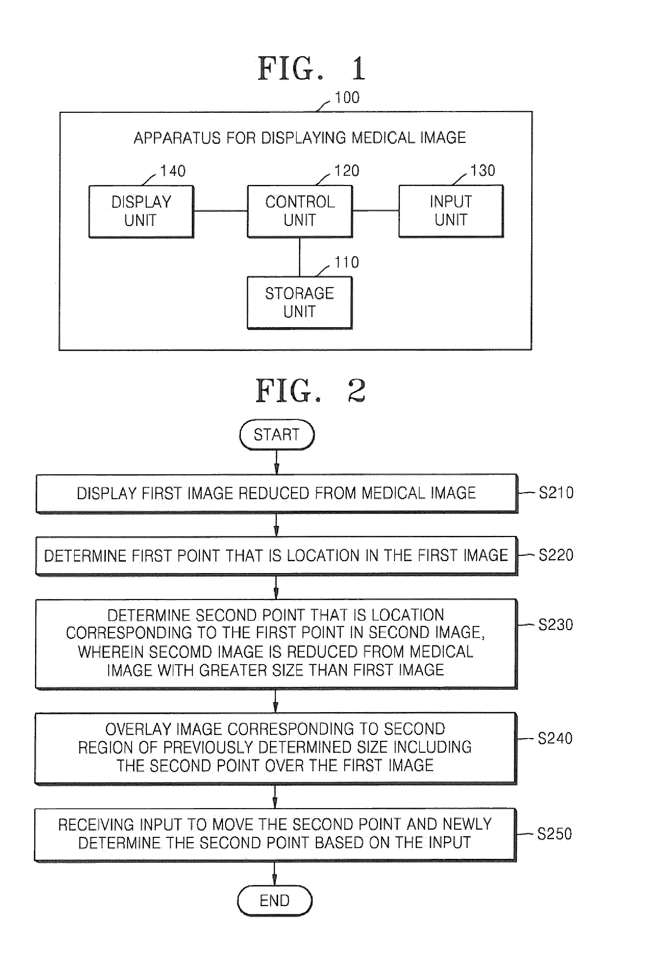

FIG. 1 is a block diagram of an apparatus 100 for displaying a medical image according to an embodiment of the present invention. Referring to FIG. 1, the apparatus 100 for displaying the medical image may include a storage unit 110, a control unit 120, an input unit 130, and a display unit 140.

Elements related to the present embodiment are illustrated in FIG. 1. However, it will be understood by those of ordinary skill in the art that besides the elements illustrated in FIG. 1 other general-purpose elements may be further included.

The apparatus 100 for displaying the medical image is an apparatus for acquiring the medical image and displaying the medical image to a user. The apparatus 100 for displaying the medical image may be basically any apparatuses having a screen for displaying the medical image. For example, the apparatus 100 for displaying the medical image may be a cellular phone, a medical diagnosis apparatus, a general-purpose computer system, a laptop, a personal digital assistant (PDA), a table PC, or the like.

The storage unit 110 acquires and stores the medical image. The apparatus 100 for displaying the medical image may directly capture a subject to acquire a medical image while acquiring and storing a medical image captured by an external apparatus. The storage unit 110 may be a storage server implemented on the Web.

If the medical image is captured by the external apparatus, the storage unit 110 may acquire the medical image by using a picture archiving communication system (PACS). The storage unit 110 may also acquire the medical image via a portable storage medium like a universal serial bus (USB) or a portable hard disk. The storage unlit 110 may download and store the medical image on line via the storage server.

The control unit 120 generally controls the storage unit 110, the input unit 130, and the display unit 140. For example, the control unit 120 may receive the medical image stored in the storage unit 110 and display the medical image on the display unit 140. The control unit 120 may receive a determination signal or a marking signal from the input unit 130 to control the display unit 140.

According to an embodiment of the present invention, the control unit 120 reduces the medical image stored in the storage unit 110 at a predetermined reduction rate and transmits the reduced medical image to the display unit 140 to display the reduced medical image. A control process will be described in more detail with reference to FIG. 2.

The input unit 130 generates the determination signal related to the present embodiment. The input unit 130 transmits the determination signal to the control unit 120 so that the control unit 120 may control the medical image displayed on the display unit 140 according to the determination signal. This will be described in detail with reference to FIG. 2.

The input unit 130 may use a mouse or a track ball to generate the determination signal. Alternatively, the input unit 130 may use a touch screen having a layer structure with the display unit 140. According to another embodiment, the input unit 130 may receive the determination signal from a user input receiving device connected to the outside of the apparatus 100 for displaying the medical image.

The display unit 140 displays the medical image stored in the storage unit 110. A structure of the display unit 140 may include one of a liquid crystal display (LCD), an organic light emitting diode (OLED), a flexible display, and a 3D display but is not limited thereto. The display unit 140 may have any structures by which the medical image may be displayed thereon.

According to an embodiment, if the display unit 140 is the touch screen having the layer structure with the input unit 130 as described above, the display unit 140 may simultaneously act as the input unit 130.

If the display unit 140 displays the medical image, the display unit 140 may reduce the medical image at a predetermined reduction rate to display the reduced medical image due to a pixel and size of the display unit 140. Accordingly, if a measurement operation like a caliper is performed on the reduced medical image, a medical diagnosis system may acquire an inaccurate value with respect to the reduction rate.

For example, in a case where the display unit 140 reduces a medical image having a size of 800x600 to 1/4 to display the medical image, if a user selects a point of the reduced medical image, the selected point automatically selects a value of a point from among points of 4x4=16 of the original medical image before being reduced. The more the image is reduced, the more the error increases. Thus, it is required to improve accuracy in diagnosing a subject using a medical image.

A method of displaying a medical image using the construction of the apparatus 100 for displaying the medical image will now be described with reference to FIGS. 2 through 6.

FIG. 2 is a flowchart of a method according to an embodiment of the present invention. The method shown in FIG. 2 includes time-series operations performed by the apparatus 100 for displaying the medical image, the storage unit 110, the control unit 120, the input unit 130, and the display unit 140 shown in FIG. 1. Thus, the descriptions with reference to the elements shown in FIG. 1 are also applicable to the method of FIG. 2 although omitted below.

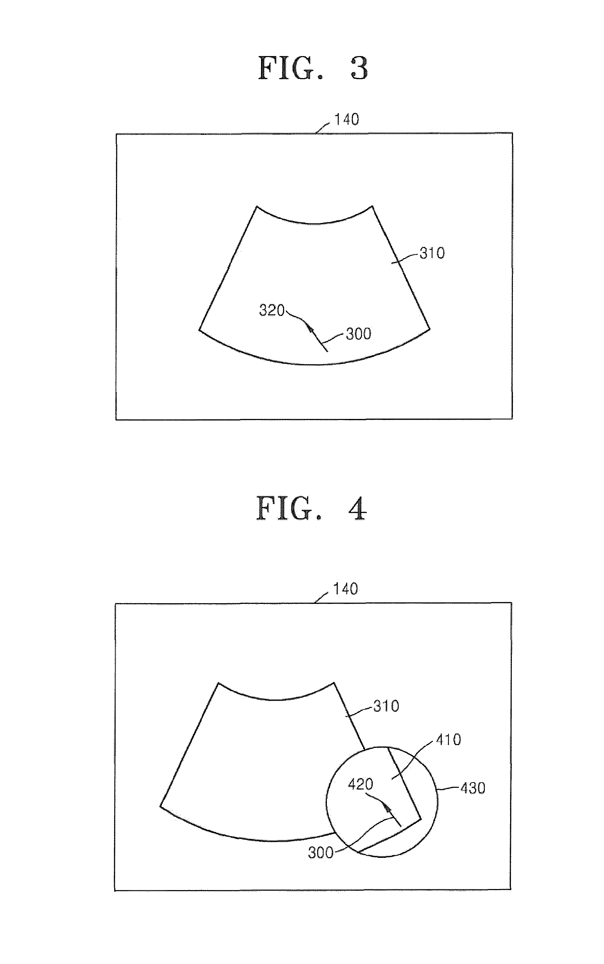

FIGS. 3 through 6 will be described with FIG. 2. FIG. 2 is a flowchart of a method of displaying a medical image, according to an embodiment of the present invention.

In operation S210, the display unit 140 displays a first image 310 reduced from the medical image. In general, the display unit 140 reduces the medical image at a predetermined reduction rate to display the reduced medical image due to resolution and size thereof. The reduced medical image is referred to as the first image 310.

For more details on operation S210, the control unit 120 may receive the medical image from the storage unit 110. Thus, the control unit 120 may determine the predetermined reduction rate with respect to the resolution and size of the display unit 140. If the predetermined reduction rate is determined, the control unit 120 reduces the medical image at the predetermined reduction rate, generates the first image 310, and transmits the first image 310 to the display unit 140. Thus, the display unit 140 displays the first image 310.

Although the display unit 140 displays one first image 310 in the present embodiment, the display unit 140 may display two or more first images 310 according to settings.

In operation S220, the control unit 120 determines a first point 320 that is one of locations of the first image 310. For example, referring to FIG. 3, the control unit 120 determines a location indicating an edge of a pointer 300 displayed on the display unit 140 as the first point 320.

According to an embodiment, the control unit 120 may use a determination signal for determining the first point 320 generated by the input unit 130 to determine the first point 320. The input unit 130 may use a mouse, a track ball, or a touch screen from a user to generate the determination signal for determining the first point 320.

In the present embodiment, the user may personally manipulate the pointer 300 displayed on the display unit 140 to generate the determination signal for determining the first point 320, If the input unit 130 is the touch screen, the display unit 140 may simultaneously act as the input unit 130.

Operation S230 will be described with reference to FIGS. 3 and 4. In operation S230, the control unit 120 determines a second point 420 that is a location corresponding to the first point 320 in a second image 410, in which the second image 410 is reduced from the medical image having a size greater than that of the first image 310.

As described with reference to the first image 310, the control unit 120 reduces the medical image stored in the storage unit 110 at a predetermined reduction rate to generate the second image 410. However, the second image 410 is greater than the first image 310. For example, if the control unit 120 reduces the first image 310 to 30% of the medical image, the second image 410 is reduced to 70% of the medical image. This is because a location can be more accurately selected from the second image 410 greater than the first image 310. The related effect of the present invention will be in detail described later.

The control unit 120 transmits the second image 410 to the display unit 140. The display unit 140 does not directly display the second image 410 unlike the first image 310. An operation of displaying the second image 410 will be in more detail described with reference to operation S240.

The control unit 120 reduces the medical image at the predetermined reduction rate in operation S210. The control unit 120 determines the first point 320 and then determines the second point 420 based on a predetermined reduction rate (hereinafter referred to as a "first rate") used to generate the first image 310 and another predetermined reduction rate (hereinafter referred to as a "first rate") used to generate the second image 410. The second point 420 is a standard point for displaying the second image 410 on the display unit 140.

For example, the control unit 120 determines a point corresponding to the first point 320 in the original medical image before being reduced based on the first rate. Thereafter, the control unit 120 may determine the second point 420 of the second image 410 from the determined point in the original medical image before being reduced based on the second rate. As another example, the control unit 120 may directly determine the second point 420 of the second image 410 corresponding to the first point 320 of the first image 310 based on both the first rate and the second rate.

The second point 420 is more accurate than the first point 320 in determining a specific location in the medical image. The second image 410 is greater than the first image 310 and thus the second point 420 is determined with a lower error value than that in performing a measurement process using the first point 320. Such an improved method of displaying the medical image can be used to efficiency perform the measurement process on the medical image. In this regard, the greater the difference between the first rate and the second rate, the more efficiently the measurement process is performed. In other words, the greater the difference between the size of the first image 310 and the size of the second image 410, the more efficiently the measurement process is performed.

According to the present embodiment, it is closer to an actual measurement value to determine the second point 420 in the second image 140 having a size of 800x600 than to determine the first point 320 in the first image 310 having a size of 200x150. Thus, the apparatus 100 for displaying the medical image uses the second point 420 internally, thereby a diagnose system is able to accurately diagnose a subject.

According to another embodiment, the control unit 120 determines the second point 420 corresponding to the first point 320 in the original medical image other than the second image 410. In other words, the control unit 120 determines the second image 420 in the original medical image that is not reduced. The original medical image is greater than the second image 410, and thus the medical diagnosis system can perform more accurate measurement process.

According to another embodiment, if the reduced size of the first image 310 is greater than a predetermined size, the control unit 120 does not reduce the medical image but may acquire the second image 410 that is rather enlarged in order to acquire an accurate measurement point. Thus, the control unit 120 may determine the second point 420 in the second image 410 enlarged from the original medical image.

In operation S240, the display unit 140 overlays the second image 410 corresponding to a second region 430 having a previously determined size and including the second point 420 over the first image 310. The second region 430 may have a circular shape having a diameter of a previously determined length with respect to the second point 420. The second region 430 may have a variety of shapes like a rectangular shape other than the circular shape. A size of the second region 430 is previously determined by the control unit 120. The control unit 120 may allocate a certain percent or rate of the size of the display unit 140 to the second region 430.

The display unit 140 overlays the second image 410 corresponding to the second region 430 transmitted from the control unit 120 over the first image 310. Thus, a user of the apparatus 100 for displaying the medical image may determine an accurate location in the first image 310 by using the overlaid second image 410.

According to another embodiment, with reference to FIG. 4, the display unit 140 may overlay the second image 410 corresponding to the second region 430 over a first region (not shown) including the first point 320. In this case, a size of the first region (not shown) may be equal to a size of the second region 430.

According to the embodiment with reference to FIG. 4, the first point 320 may be a location pointed out by the pointer 300 according to the determination signal received from the input unit 130. The second point 420 corresponding to the first point 320 determined by the control unit 120 and the first point 320 belong to images having different sizes but have the same location in the images. Thus, if the display unit 140 overlays the second image 410 corresponding to the second region 430 over the first region (not shown), the second region 430 is displayed on the display unit 140 with respect to the first point 320 and the second point 420.

In operation S250, the control unit 120 receives an input to move the second point 420, and newly determines the second point 420 based on the received input. The input unit 130 may generate the input to move the second point 420. Alternatively, as described above, the input unit 130 may receive an input to move the second point 420 from the user and transmit the input to the control unit 120. For example, the user may enter the input to move the second point 420 using a mouse, a track ball, or a touch screen.

The control unit 120 receives the input to move the second point 420 from the input unit 130, and newly determines the second point 420 based on the received input. Thus, the control unit 120 may measure an accurate location to be measured by the user in the second image 410. The newly determined second point 420 may be used for an actual measurement operation, and have higher accuracy than the first point 320, and thus the user can efficiently diagnose the subject.

According to an embodiment, in addition to operations S210 to S250 described above, the method of displaying the medical image may further include an operation of the control unit 120 for newly determining the first point 320 according to the newly determined second point 420, and an operation of the display unit 140 for overlaying an image corresponding to the second region 430 including the newly determined second point 420 over a first region (not shown) including the newly determined first point 320. According to the present embodiment, the display unit 140 may display the second region 430 including the newly determined second point 420 to efficiently diagnose a point in the medical image to be measured by the user.

According to another embodiment, the control unit 120 may store the newly determined second point 420 in the storage unit 110. In the present embodiment, the second point 420 stored in the storage unit 110 may be retrieved as occasion arises inside the apparatus 100 for displaying the medical image.

According to another embodiment, the display unit 140 marks the first point 320 in the first image 310. The input unit 130 generates a marking signal and transmits the marking signal to the control unit 120. The control unit 12 transfers the marking signal to the display unit 140. The display unit 140 marks a marker 510 on the first point 320 based on the marking signal.

As described above, since the control unit 120 determines the second point 420, a measurement system performs an accurate measurement process using the second point 420. However, the display unit 140 marks the marker 510 in the first point 320 of the first image 310 corresponding to the second point 420. Thus, although the display unit 140 seems to externally perform the measurement process by using the first point 320, the measurement system may internally perform the accurate measurement process by using the second point 420.

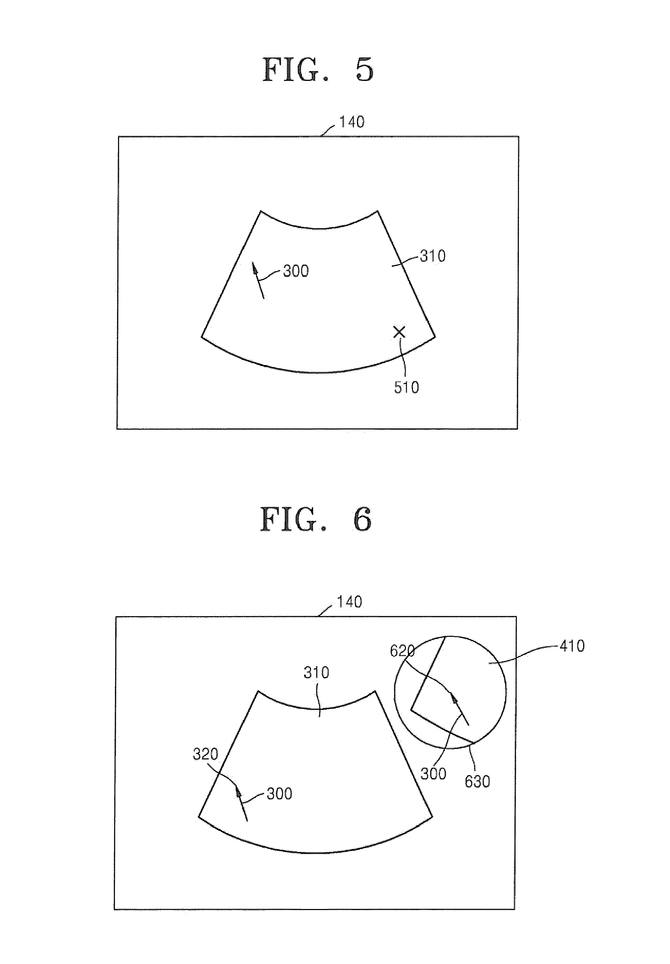

FIG. 6 illustrates a medical image displayed on the display unit 140 of the apparatus 100 for displaying the medical image, according to another embodiment of the present invention.

The first image 310 displayed on the display unit 140 includes display points 620 present in a plurality of previously determined locations. The display unit 140 overlays the second image 410 corresponding to the second region 430 over a display region 630 including one of the display points 620 farthest away from the first point 320.

Assuming that the display unit 140 has a rectangular shape, the four display points 620 may be in the first region 310 near angular points of the display unit 140. Referring to FIG. 6, the first point 320 pointed out by the pointer 300 is at left and below the first image 310. Thus, the display point 620 farthest away from the first point 320 is at right and above the first image 310. Furthermore, the display unit 140 overlays the second image 410 corresponding to the second region 430 over the display region 630 including the display point 620 at right and above the first image 310.

In the present embodiment, the control unit 120 may use a touch screen to determine the second point 420 when a part of the display unit 140 is hidden by a user's finger. If the input unit 130 uses the touch screen to generate a determination signal for determining the second point 420, it is inconvenient to read the second image 410 included in the second region 430 due to the user's finger. In the present embodiment, the display unit 140 overlays the second image 410 corresponding to the second region 430 over the display region 630 other than the second region 430, and thus the user can determine a location of the second point 420 in the second image 410 irrespective of a size of the display unit 140.

As described above, a medical diagnosis system may acquire an accurate measurement value of a medical image displayed on an apparatus for displaying the medical image because of a reduced screen of the apparatus. A user may efficiently analyze and read the medical image because of a reduced error of the measurement value of the medical image.

The embodiments of the present invention may be written as computer programs and can be implemented in general-use digital computers that execute the programs using a computer readable recording medium. In addition, a structure of data used in the above-described method may be recorded in a computer readable recording medium through various methods. Examples of the computer readable recording medium include magnetic storage media (e.g., ROMs, RAMS, universal serial buses (USBs), floppy disks, hard disks, etc.), optical recording media (e.g., CD-ROMs, or DVDs), and storage media such as PC interfaces (e.g., PCI, PCI-express, WiFi, etc.).

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A method of displaying a medical image, the method comprising:
displaying a first image reduced from the medical image;
determining a first point that is a location in the first image;
determining a second point that is a location corresponding to the first point in a second image, wherein the second image is reduced from the medical image having a size greater than that of the first image;
overlaying an image corresponding to a second region of a previously determined size including the second point over the first image; and
receiving an input to move the second point and newly determining the second point based on the input.

2. The method of claim 1, wherein the overlaying comprises: overlaying the image corresponding to the second region over a first region including the first point of the first image.

3. The method of claim 1, further comprising:
newly determining the first point according to the newly determined second point; and
overlaying an image corresponding to a new second region including the newly determined second point over a new first region including the newly determined first point.

4. The method of claim 1, further comprising: marking the newly determined first point in the first image.

5. The method of claim 1, further comprising: storing the newly determined second point.

6. The method of claim 1, wherein the first image comprises a plurality of display points present in a plurality of previously determined locations,
wherein the overlaying comprises: overlaying the image corresponding to the second region over a display region including one of the plurality of display points farthest away from the first point.

7. A method of displaying a medical image, the method comprising:
displaying a reduction image reduced from the medical image;
determining a first point that is a location in the reduction image;
determining a second point that is a location corresponding to the first point in the medical image;
overlaying an image corresponding to a second region of a previously determined size including the second point over the reduction image; and
receiving an input to move the second point and newly determining the second point based on the input.

8. An apparatus for displaying a medical image, the apparatus comprising:
a display unit for displaying a first image reduced from the medical image;
a control unit for a determining a first point that is a location in the first image, and determining a second point that is a location corresponding to the first point in a second image, wherein the second image is reduced from the medical image having a size greater than that of the first image; and
an input unit for receiving an input to move the second point,
wherein the display unit overlays an image corresponding to a second region of a previously determined size inclucing the second point over the first image, and
wherein the control unit newly determines the second point based on the input.

9. The apparatus of claim 8, wherein the display unit overlays the image corresponding to the second region over a first region including the first point of the first image.

10. The apparatus of claim 8, wherein the control unit newly determines the first point according to the newly determined second point, and
wherein the display unit overlays an image corresponding to a new second region including the newly determined second point over a new first region including the newly determined first point.

11. The apparatus of claim 8, wherein the display unit marks the newly determined first point in the first image.

12. The apparatus of claim 8, further comprising: a storage unit for storing the newly determined second point.

13. The apparatus of claim 8, wherein the first image comprises a plurality of display points present in a plurality of previously determined locations,
wherein the display unit overlays the image corresponding to the second region over a display region including one of the plurality of display points farthest away from the first point.

14. An apparatus for displaying a medical image, the apparatus comprising:
a display unit for displaying a reduction image reduced from the medical image;
a control unit for determining a first point that is a location in the reduction image, and determining a second point that is a location corresponding to the first point in the medical image; and
an input unit for receiving an input to move the second point,
wherein the display unit overlays an image corresponding to a second region of a previously determined size including the second point over the reduction image, and
wherein the control unit newly determines the second point based on the input.

15. A computer readable recording medium having recorded thereon a program for executing the method of claim 1.
